(19) Europäisches Patentamt
European Patent Office
Office européen des brevets

(11) EP 3 604 314 B1

(12) **EUROPEAN PATENT SPECIFICATION**

(45) Date of publication and mention of the grant of the patent:
**23.02.2022 Bulletin 2022/08**

(21) Application number: **18776514.4**

(22) Date of filing: **22.03.2018**

(51) International Patent Classification (IPC):
***C07D 493/04*** *(2006.01)* ***C07C 61/08*** *(2006.01)*
***C07C 51/56*** *(2006.01)*

(52) Cooperative Patent Classification (CPC):
**C07D 493/04**

(86) International application number:
**PCT/JP2018/011333**

(87) International publication number:
**WO 2018/180855 (04.10.2018 Gazette 2018/40)**

(54) **METHOD FOR PRODUCING 1,2,4,5-CYCLOHEXANETETRACARBOXYLIC DIANHYDRIDE**

VERFAHREN ZUR HERSTELLUNG VON 1,2,4,5-CYCLOHEXANTETRACARBONSÄURE-DIANHYDRID

PROCÉDÉ DE PRODUCTION DE DIANHYDRIDE DE 1,2,4,5-CYCLOHEXANETÉTRACARBOXYLIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.03.2017 JP 2017065899**
**29.03.2017 JP 2017065907**

(43) Date of publication of application:
**05.02.2020 Bulletin 2020/06**

(73) Proprietor: **Mitsubishi Gas Chemical Company, Inc.**
**Tokyo 100-8324 (JP)**

(72) Inventors:
- **SHIRAI, Shinyo**
**Kurashiki-shi**
**Okayama 712-8525 (JP)**
- **KUMANO, Tatsuyuki**
**Kurashiki-shi**
**Okayama 712-8525 (JP)**
- **SAITO, Shinya**
**Kurashiki-shi**
**Okayama 712-8525 (JP)**

(74) Representative: **Hoffmann Eitle**
**Patent- und Rechtsanwälte PartmbB**
**Arabellastraße 30**
**81925 München (DE)**

(56) References cited:
**EP-A1- 1 323 700**
**CN-A- 103 992 330**
**JP-A- H09 328 538**
**JP-A- S53 103 437**
**JP-A- S53 103 437**
**JP-A- 2003 286 222**
**JP-A- 2006 045 166**
**JP-A- 2006 124 313**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### Technical Field

The present invention relates to a method for producing 1,2,4,5-cyclohexanetetracarboxylic dianhydride.

### Background Art

Alicyclic acid anhydrides have been used as starting materials for functional polyimides and functional epoxy resins. Among such acid anhydrides, 1,2,4,5-cyclohexanetetracarboxylic dianhydride is used as a starting material for polyimide resins that exhibit particularly good heat resistance, solvent solubility and thermoplasticity as well as low water absorbability, dimensional stability and the like.

For the synthesis of a polyimide, an acid anhydride and a diamine are desirably used in equivalent amounts because the molecular weight of the polyimide does not sufficiently increase when the molar balance of the acid anhydride and the diamine is lost. Also, impurities contained in the diamine and the acid anhydride cause the loss of molar balance. Accordingly, the acid anhydride as a starting material is required to have a high purity.

An acid anhydride is known to be obtained by subjecting a hydrogenated aromatic polycarboxylic acid to a dehydration reaction. For example, 1,2,4,5-cyclohexanetetracarboxylic dianhydride is produced by cyclodehydration of 1,2,4,5-cyclohexanetetracarboxylic acid. A method involving a heat treatment or a method involving a dehydrating agent is commonly used to synthesize a cyclic acid anhydride by dehydrating and ring-closing the carboxy groups adjacently bonded to the 6-membered ring of a hydrogenated aromatic polycarboxylic acid. An acid anhydride such as acetic anhydride or propionic anhydride is used as a dehydrating agent.

A method involving thermal reflux using acetic anhydride is known as a method for cyclodehydrating 1,2,4,5-cyclohexanetetracarboxylic acid (see PTL1).

### Citation List

### Patent Literature

PTL1: JP 2003-286222 A
PTL2: EP 1 323 700 A
PTL3: JP S53103437 A

### Summary of Invention

### Technical Problem

PTL 1 discloses that a hydrogenated aromatic polycarboxylic acid is dehydrated by a method involving acetic anhydride as a dehydrating agent, but the method has the following problem: the high dehydration rate set forth in PTL 1 cannot be reproduced depending on the conditions.

PTL 2 relates to a process for producing a hydrogenated aromatic polycarboxylic acid in which an aromatic ring of an aromatic polycarboxylic acid is hydrogenated and a process for producing a hydrogenated aromatic polycarboxylic anhydride.

PTL 3 is directed to the feeding of the slurry of terephthalic acid and ethylene glycol continuously and smoothly into a pressed esterification reactor maintaining the pressure which is above the reactor pressure and below the discharge pressure.

An object of the present invention is to provide a method for producing 1,2,4,5-cyclohexanetetracarboxylic dianhydride, which is capable of stably achieving a high dehydration rate.

### Solution to Problem

As a result of having conducted diligent research to solve the above problem, the present inventors found that the dehydration rate is improved by feeding 1,2,4,5-cyclohexanetetracarboxylic acid in a continuous manner to a dehydrating agent, and accomplished the present invention. The present invention provides [1] to [3] below.

[1] A method for producing 1,2,4,5-cyclohexanetetracarboxylic dianhydride by subjecting 1,2,4,5-cyclohexanetetracarboxylic acid to a dehydration reaction in a slurry state in the presence of a dehydrating agent, wherein;

the dehydration agent is acetic anhydride;
the acetic anhydride is used in an amount of 2.0 to 100 moles per mole of the 1,2,4,5-cyclohexanetetracarboxylic acid; and
the dehydration reaction of the 1,2,4,5-cyclohexanetetracarboxylic acid is carried out in the presence of the dehydrating agent and a solvent, wherein
the solvent is acetic acid, characterized in that the 1,2,4,5-cyclohexanetetracarboxylic acid is fed in a continuous manner to the dehydrating agent, wherein an average particle size of the 1,2,4,5-cyclohexanetetracarboxylic acid is less than 20 μm.

[2] The method according to [1], wherein an average particle size of the 1,2,4,5-cyclohexanetetracarboxylic acid is less than 7 μm.
[3] The method according to [1] or [2], wherein a reaction temperature of the dehydration reaction is 80 to 150°C.

Advantageous Effects of Invention

According to the present invention, a method for producing 1,2,4,5-cyclohexanetetracarboxylic dianhydride, which is capable of stably achieving a high dehydration rate, can be provided.

Description of Embodiments

The present invention will be described in reference to embodiments below. In the following description, "A to B" indicating a numerical range denotes "A or more and B or less" (in the case of A < B) or "A or less and B or more" (in the case of A > B). That is, "A to B" denotes a numerical range including end points A and B.
Also, part by mass and % by mass are synonymous with part by weight and % by weight, respectively.
The method for producing 1,2,4,5-cyclohexanetetracarboxylic dianhydride (hereinafter also simply referred to as "cyclohexanetetracarboxylic dianhydride") of the present invention is a method for producing 1,2,4,5-cyclohexanetetracarboxylic dianhydride by subjecting 1,2,4,5-cyclohexanetetracarboxylic acid (hereinafter also simply referred to as "cyclohexanetetracarboxylic acid") to a dehydration reaction in a slurry state in the presence of a dehydrating agent, wherein the 1,2,4,5-cyclohexanetetracarboxylic acid is fed in a continuous manner to the dehydrating agent, the dehydration agent is acetic anhydride, the acetic anhydride is used in an amount of 2.0 to 100 moles per mole of the 1,2,4,5-cyclohexanetetracarboxylic acid, and the dehydration reaction of the 1,2,4,5-cyclohexanetetracarboxylic acid is carried out in the presence of the dehydrating agent and a solvent, wherein the solvent is acetic acid.
As a result of having conducted diligent research, the inventors found that a high dehydration rate can be stably obtained by feeding 1,2,4,5-cyclohexanetetracarboxylic acid in a continuous manner to the reaction system, and accomplished the present invention.
In the following description, the dehydrating agent and the solvent may be collectively referred to as a "solution."
As a result of having conducted research, the inventors found that such an effect is significant when the average particle size of the starting material cyclohexanetetracarboxylic acid is as small as less than 20 μm.
In the reaction system, either the cyclohexanetetracarboxylic acid as a starting material or the cyclohexanetetracarboxylic dianhydride as a product is not completely dissolved in a solution at least in the final stage of the reaction. Accordingly, the reaction proceeds in a slurry state at least when the addition of the starting material is finished.
It is considered that the dehydration reaction of cyclohexanetetracarboxylic acid proceeds in a state where cyclohexanetetracarboxylic acid is dissolved in a dehydrating agent and a solvent (solution), and cyclohexanetetracarboxylic dianhydride is produced. It is considered that, at this time, the reaction proceeds in a slurry state, and thus the produced cyclohexanetetracarboxylic dianhydride precipitates while incorporating cyclohexanetetracarboxylic acid present in the solution. That is, dehydration (a dehydration reaction) and a crystal precipitation reaction progress at the same time. It is inferred that, in this instance, the concentration of cyclohexanetetracarboxylic acid in the solution increases especially when the particle size of the cyclohexanetetracarboxylic acid as a starting material is small, thus the cyclohexanetetracarboxylic acid as a starting material is incorporated and precipitates when the cyclohexanetetracarboxylic dianhydride as a product undergoes crystal precipitation, and accordingly the dehydration rate is impaired.
It is conjectured that in the present invention, by adding the cyclohexanetetracarboxylic acid as a starting material in a continuous manner, the cyclohexanetetracarboxylic acid concentration in the reaction system is suppressed, as a result, the amount of cyclohexanetetracarboxylic acid incorporated into the cyclohexanetetracarboxylic dianhydride as a product is suppressed, and a high dehydration rate is stably obtained.
In the method for producing cyclohexanetetracarboxylic dianhydride of the present invention, the following

dehydration reaction occurs.

[Formula 1]

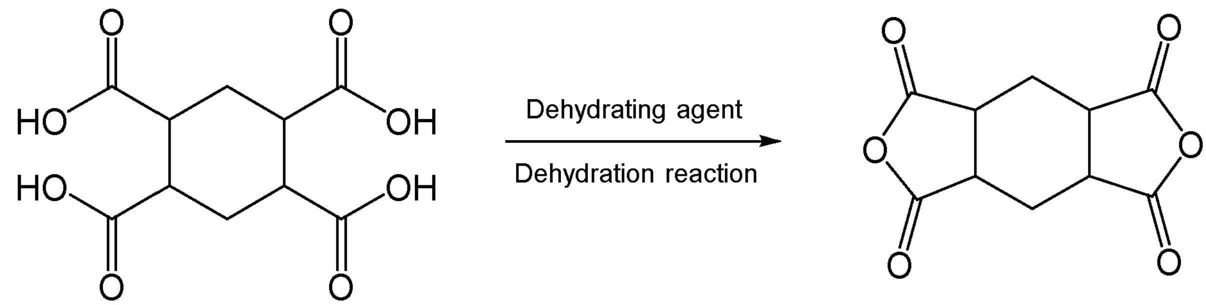


1,2,4,5-Cyclohexanetetracarboxylic acid

1,2,4,5-Cyclohexanetetracarboxylic dianhydride

<1,2,4,5-Cyclohexanetetracarboxylic acid>

In the present invention, 1,2,4,5-cyclohexanetetracarboxylic acid is not particularly limited. A commercially available product may be purchased, or 1,2,4,5-cyclohexanetetracarboxylic acid may be produced by nuclear hydrogenation of pyromellitic acid.

The method for producing 1,2,4,5-cyclohexanetetracarboxylic acid by nuclear hydrogenation of pyromellitic acid is not particularly limited. Examples include a method in which pyromellitic acid is dissolved or suspended in a reaction solvent and hydrogenated in the presence of a catalyst at a hydrogen partial pressure of 1.0 to 15 MPa at a reaction temperature of 30 to 80°C wherein a supported catalyst containing rhodium as well as palladium and/or platinum supported on a carbon carrier is used in a specific amount as the catalyst, as described in WO 2010/010869; and a method in which pyromellitic acid is hydrogenated at a hydrogen partial pressure of 1 MPa or more in the presence of a catalyst containing a noble metal composed of rhodium or palladium or both in a proportion of 0.5 to 10 parts by mass per 100 parts by mass of pyromellitic acid, as described in PTL1.

After the nuclear hydrogenation reaction, for example, the catalyst is separated by filtration at a temperature similar to the reaction temperature, the filtrate is cooled to room temperature, the precipitated solids are separated by filtration, the filtered solids are dried, and thereby 1,2,4,5-cyclohexanetetracarboxylic acid can be obtained. Also, the reaction solvent is distilled off from the filtrate to concentrate the filtrate, the precipitated solids are separated by filtration, then the hydrogenated product of pyromellitic acid is crystallized by being cooled, concentrated, the crystals thereof are subjected to solid-liquid separation, and thereby high-purity 1,2,4,5-cyclohexanetetracarboxylic acid can be obtained.

The present inventors found that, as in the present invention, a good dehydration rate can be obtained when a starting material having a small average particle size is used by feeding cyclohexanetetracarboxylic acid in a continuous manner. Accordingly, the dehydration rate improving effect attained when the cyclohexanetetracarboxylic acid as a starting material is fed in a continuous manner as in the present invention is more significant when the starting material has a smaller average particle size. According to the present invention, the dehydration rate improving effect is achieved when the average particle size of the starting material 1,2,4,5-cyclohexanetetracarboxylic acid is less than 20 μm, significant when less than 15 μm, more significant when less than 10 μm, and even more significant when less than 7 μm.

Here, as for the average particle size of cyclohexanetetracarboxylic acid, the lengths of the major axes of 100 particles on a 100x or 1000x image taken by a field emission-scanning electron microscope (FE-SEM) are measured using image processing software Image J. The average value of the resulting major axis lengths of the particles is regarded as the average particle size of cyclohexanetetracarboxylic acid.

In the present invention, it is preferable to introduce a dehydrating agent and a solvent into a reaction vessel in advance and feed cyclohexanetetracarboxylic acid thereto in a continuous manner.

<Dehydrating agent>

The dehydrating agent used in the present invention is acetic anhydride from the viewpoint of economy and usability.

In the present invention, acetic anhydride is used in an amount of 2.0 to 100 moles per mole of 1,2,4,5-cyclohexanetetracarboxylic acid.

From the viewpoint of obtaining a sufficient dehydration rate, acetic anhydride is used in an amount of 2.0

moles or more, preferably 2.5 moles or more, and more preferably 3 moles or more, and from the viewpoint of economy and from the viewpoint of removing the dehydrating agent after reaction, acetic anhydride is used in an amount of 100 moles or less, preferably 75 moles or less, more preferably 50 moles or less, further preferably 25 moles or less, and furthermore preferably 5 moles or less.

In the present invention, acetic anhydride used as a dehydrating agent is a liquid and thus also functions as a solvent.

<Dehydration reaction conditions>

In the present invention, cyclohexanetetracarboxylic acid is subjected to a dehydration reaction (also referred to as an anhydration reaction) in a slurry state in the presence of a dehydrating agent. The slurry state means that the cyclohexanetetracarboxylic acid as a starting material does not completely dissolve in a dehydrating agent and a solvent and partially exists in a solid state and, also, the produced acid anhydride does not completely dissolve in the dehydrating agent and the solvent and partially exists in a solid state. Accordingly, a state where either the starting material or the product or both partially exist in a solid state in the reaction system is referred to as a slurry state.

In the present invention, it is sufficient that at least part of the dehydration reaction is carried out in a slurry state. This is not to exclude an embodiment in which the cyclohexanetetracarboxylic acid as a starting material and the cyclohexanetetracarboxylic dianhydride as a product are completely dissolved in the initial stage of feeding the starting material.

From the viewpoint of promoting dissolution of cyclohexanetetracarboxylic acid in a solvent to promote the dehydration reaction of cyclohexanetetracarboxylic acid, the reaction temperature of the dehydration reaction is preferably 80°C or more, more preferably 90°C or more, and even more preferably 95°C or more. From the viewpoint of suppressing the decomposition of the starting material and the product and the volatilization of a dehydrating agent and a solvent, which will be described below, and preventing the product from caking after lowering the temperature, the reaction temperature in the dehydration reaction is preferably 150°C or less, more preferably 140°C or less, even more preferably 130°C or less, and further preferably 120°C or less.

The dehydration reaction may involve only heating the slurry of cyclohexanetetracarboxylic acid and a dehydrating agent, or may involve heating the dehydrating agent to reflux.

The dehydration reaction is preferably carried out in an atmosphere of an inert gas such as nitrogen gas.

<Solvent>

In the present invention, the dehydration reaction is carried out in the presence of a dehydrating agent and a solvent.

Acetic acid (also referred to as glacial acetic acid) is used as a solvent. Acetic acid is preferably used in an amount of 0.5 to 10 times by volume and more preferably 1 to 5 times by volume based on the dehydrating agent.

In addition to acetic acid, a hydrocarbon, a halogenated hydrocarbon, an ester, a ketone, an ether, a fatty acid, having a boiling point of 50°C or more may be added as a solvent.

<Feeding of cyclohexanetetracarboxylic acid>

In the method for producing 1,2,4,5-cyclohexanetetracarboxylic dianhydride of the present invention, the cyclohexanetetracarboxylic acid as a starting material is fed in a continuous manner to the dehydrating agent.

Here, feeding in a divided or continuous manner means feeding cyclohexanetetracarboxylic acid in at least two divided portions to the dehydrating agent, or continuously feeding cyclohexanetetracarboxylic acid to the dehydrating agent. Feeding in a divided manner does not mean that the entirety of the divided portions is fed at once, but means that the divided portions are fed in multiple steps that are separated by intervals.

An embodiment in which cyclohexanetetracarboxylic acid is fed in a continuous manner and an embodiment in which cyclohexanetetracarboxylic acid is fed in a divided manner may be combined, and such a combined embodiment is also encompassed within the present invention. Specific examples are an embodiment in which part of cyclohexanetetracarboxylic acid is fed at once or in several divided portions, and then the remainder of cyclohexanetetracarboxylic acid is fed in a continuous manner; and an embodiment in which part of cyclohexanetetracarboxylic acid is fed in an continuous manner, and then the remainder is fed at once or in several divided portions.

According to the present invention where cyclohexanetetracarboxylic acid is fed in a continuous manner, the feeding rate is not particularly limited, and may be constant or may be suitably changed during feeding. In consideration of the ease of feeding, the feeding rate is preferably constant. Where the average feeding rate per 10 minutes is Y, the feeding rate per any 10 minutes is preferably 0.1 Y to 10 Y, more preferably 0.3 Y to 5 Y, and even more preferably 0.5 Y to 2 Y.

The feeding time is preferably 5 minutes to 1000 minutes, more preferably 15 minutes to 500 minutes, and even more preferably 30 minutes to 300 minutes.

After the entire amount of cyclohexanetetracarboxylic acid is fed in a continuous manner, further the reaction is preferably continued for 0.01 to 10 hours, more preferably 0.02 to 5 hours, and even more preferably 0.05 to 3 hours.

In the present invention, the state of the starting material 1,2,4,5-cyclohexanetetracarboxylic acid is not particularly limited, and the starting material 1,2,4,5-cyclohexanetetracarboxylic acid may be fed in a powder state or added in a slurry state. When added in a slurry state, the starting material 1,2,4,5-cyclohexanetetracarboxylic acid is preferably in a slurry state in a dehydrating agent and/or a solvent.

Below, as a reference example, a system will now be described in which 3 times by mole (3A moles) of acetic anhydride is used as a dehydrating agent based on A moles of 1,2,4,5-cyclohexanetetracarboxylic acid and, further, 2.5 times by volume of acetic anhydride is used as a solvent.

In said reference examples, the dehydrating agent and the solvent are charged into a reaction vessel in advance and heated, and then the starting material 1,2,4,5-cyclohexanetetracarboxylic acid is fed in an undivided or divided manner.

Acetic anhydride has a molecular weight of 102.09 (g/mol) and a density of 1.08 (g/mL, 20°C), and thus the amount of the dehydrating agent acetic anhydride charged is 3A (mol) × 102.09 (g/mol)/1.08 (g/mL) = 283.6A mL.

Accordingly, when the entire amount of 1,2,4,5-cyclohexanetetracarboxylic acid is added to the dehydrating agent and the solvent, the concentration thereof (cyclohexanetetracarboxylic acid/(acetic anhydride + acetic acid)) is 1.01 mol/L according to formula (a) below:

$$\mathrm{A\ (mol)}/\{3.5 \times 0.2836\mathrm{A}\}\ \mathrm{(L)} \approx 1.01\ \mathrm{(mol/L)} \qquad \text{(a)}$$

The relationship between the number of divided portions and the amount of cyclohexanetetracarboxylic acid for each feeding per liter of the total amount of the dehydrating agent and the solvent is as shown in Table 1 below.

Table 1

| Number of divided portions | Amount (mol/L) for each feeding (Cyclohexanetetracarboxylic acid/(Acetic anhydride + Acetic acid)) |
|---|---|
| Not divided | 1.01 |
| 2 | 0.504 |
| 3 | 0.336 |
| 5 | 0.202 |
| 10 | 0.101 |

<Dehydration rate>

In the present invention, the dehydration rate of the 1,2,4,5-cyclohexanetetracarboxylic acid as a starting material is preferably 98.0% or more, more preferably 98.5% or more, even more preferably 99.0% or more, further preferably 99.3% or more, furthermore preferably 99.5% or more, and particularly preferably 99.7% or more.

Due to the dehydration rate being within the above range, cyclohexanetetracarboxylic dianhydride having excellent purity can be obtained.

The dehydration rate is measured by the method described in the Examples.

<Step of recovering cyclohexanetetracarboxylic dianhydride>

In the present invention, it is preferable that the method further includes the step of recovering cyclohexanetetracarboxylic dianhydride (hereinafter also simply referred to as a recovery step).

After the dehydration reaction of cyclohexanetetracarboxylic acid, the reaction solution is cooled to room temperature to precipitate crystals of cyclohexanetetracarboxylic dianhydride, which are subjected to solid-liquid separation, and thereby cyclohexanetetracarboxylic dianhydride can be obtained. The use of acetic anhydride as a dehydrating agent and the use of acetic acid as a solvent result in an increased amount of precipitated crystals and are thus industrially advantageous. Crystals of cyclohexanetetracarboxylic dianhydride separated by solid-liquid separation are preferably dried in a suitable manner.

The mother liquor from which the crystals have been separated may be recycled. Whether the mother liquor should be returned to the reaction vessel for a dehydration reaction is determined according to the extent of impurity buildup in the system.

Examples

The present invention will be described in more detail by way of Examples and Comparative Examples below.

[Preparation Example 1: Synthesis of 1,2,4,5-cyclohexanetetracarboxylic acid]

First, 390.1 kg of pyromellitic acid, 2340.9 kg of water, 131.0 kg of a 5% by mass Pd-carbon powder catalyst (manufactured by N.E. Chemcat Corporation, a water-containing product, PE-type, a water content of 55% by mass), and 56.2 kg of a 5% by mass Rh-carbon powder catalyst (manufactured by N.E. Chemcat Corporation, a product wetted with water, a water content of 50% by mass) were charged into a 3.86 $m^3$ SUS316L reaction vessel equipped with a thermocouple, a stirrer, a temperature controller, and the like. While stirring the mixture, hydrogen was fed to 8 MPa, and the temperature was raised to 50°C. While retaining the pressure and the temperature, the hydrogenation reaction was continued until the molar amount of absorbed hydrogen was 3 times the molar amount of pyromellitic acid charged. The resulting reaction solution was discharged, the catalyst was separated by filtration, and thus a colorless, transparent filtrate was obtained.

Thereafter, the resulting filtrate was concentrated until the concentration of nuclear-hydrogenated pyromellitic acid was 33% by mass, and then cooled to 20°C to precipitate crystals of 1,2,4,5-cyclohexanetetracarboxylic acid. The precipitated crystals were separated by filtration.

The resulting wet crystals of 1,2,4,5-cyclohexanetetracarboxylic acid were charged into a Flash Jet Dryer (manufactured by Seishin Enterprise Co., Ltd.), and dried under conditions having a starting material feeding rate of 55 kg/h, an inlet temperature of 170°C, an outlet temperature of 110°C, a starting material temperature of 12.4°C, a discharge air volume of 6.8 $Nm^3$/min, and a discharge pressure of 53 kPa, and thereby white crystals of 1,2,4,5-cyclohexanetetracarboxylic acid were obtained.

The average particle size of the resulting white crystals of 1,2,4,5-cyclohexanetetracarboxylic acid was 6.9 μm.

The resulting white crystals were subjected to an esterification treatment and then a gas chromatography analysis, and thus the purity of 1,2,4,5-cyclohexanetetracarboxylic acid was 89.0%.

<Measurement of average particle size>

The lengths of the major axes of particles on a 100x or 1000x image taken by FE-SEM (manufactured by Hitachi High-Technologies Corporation, S-3000N, voltage of 10 kV) were measured using image processing software Image J. The major axis lengths of 100 particles were measured, and the average value of the obtained results was regarded as the average particle size of cyclohexanetetracarboxylic acid.

[Examples 1 to 3 (reference examples) and Comparative Example 1]

First, 55.4 g (0.542 mol, 3.0 moles per mole of the entirety of cyclohexanetetracarboxylic acid added) of acetic anhydride, and 134.6 g (2.5 times the volume of acetic anhydride) of acetic acid were charged into a 500 mL four-neck glass flask equipped with a thermocouple, a Dimroth condenser, and a stirrer, and the system was replaced with nitrogen gas while stirring the mixture. Subsequently, the temperature was raised to 100°C while allowing nitrogen gas to flow at 100 mL/min, then cyclohexanetetracarboxylic acid having an average particle size of 6.9 μm obtained in Preparation Example 1 was added such that the reaction time and the number of additions were as shown in Table 2 below. The total amount added was 47.5 g (0.18 mol), and the reaction was continued 120 minutes. After the reaction, the temperature was lowered to room temperature to precipitate crystals, and then the crystals were separated. The resulting crystals were rinsed with 13.1 g of acetic anhydride and then dried to measure the dehydration rate.

The dehydration reaction carried out in the Examples was as follows. The results are shown in Table 2 below.

[Formula 2]

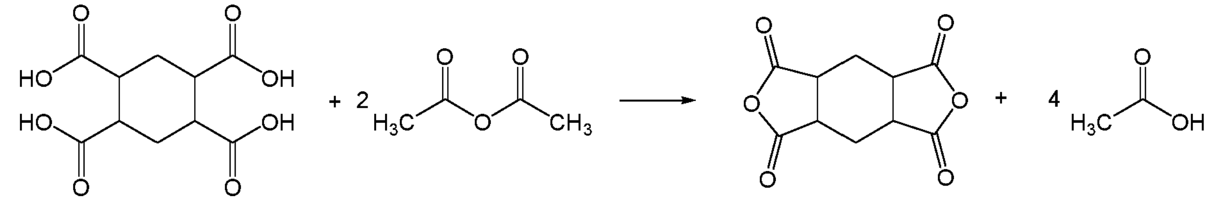

1,2,4,5-Cyclohexanetetracarboxylic acid — Acetic anhydride — 1,2,4,5-Cyclohexanetetracarboxylic dianhydride — Acetic acid

[Example 4]

First, 49.5 g of acetic anhydride and 63.1 g of acetic acid were charged into a 500 mL four-neck glass flask equipped with a thermocouple, a Dimroth condenser, and a stirrer, the temperature was raised to 100°C, and after the solution temperature reached 100°C, the feeding of a separately prepared slurry was started. The time when the feeding of the slurry was started was regarded as the reaction start time, and the slurry was continuously fed over 133 minutes at a constant rate. After the feeding of the slurry was terminated, heating and stirring were continued for 30 minutes. After the feeding of the slurry was terminated, the feeding of nitrogen gas (100 mL/min) was started. Heating was stopped 30 minutes later, and the mixture was cooled by air while continuing stirring. Three hours after heating was stopped, solid-liquid separation was carried out by suction filtration, and the solids were rinsed with 13.1 g of acetic anhydride and then dried to measure the dehydration rate.

The slurry was prepared by the following method. Specifically, 47.5 g of 1,2,4,5-cyclohexanetetracarboxylic acid obtained in Preparation Example 1, 7.8 g of acetic anhydride, and 103.0 g of acetic acid were charged into a recovery flask and stirred at room temperature to form a slurry.

Table 2

| | | Example 1* | Example 2* | Example 3* | Example 4 | Comparative Example 1 |
|---|---|---|---|---|---|---|
| Number of divided portions | | Two divided portions | Five divided portions | Ten divided portions | Continuous feeding | Not divided |
| Reaction time (min) | 0 | 50% | 20% | 10% | Constant rate feeding (continuously fed for 133 minutes) | 100% |
| | 10 | | | 10% | | |
| | 20 | | 20% | 10% | | |
| | 30 | | | 10% | | |
| | 40 | | 20% | 10% | | |
| | 50 | | | 10% | | |
| | 60 | 50% | 20% | 10% | | |
| | 70 | | | 10% | | |
| | 80 | | 20% | 10% | | |
| | 90 | | | 10% | | |
| Dehydration rate (%) | | 98.30 | 99.70 | 99.89 | >99.9 | 96.68 |
| *reference examples | | | | | | |

<Measurement of dehydration rate>

As for the dehydration rate of cyclohexanetetracarboxylic acid, a sample was analyzed by liquid chromatography to quantify the 1,2,4,5-cyclohexanetetracarboxylic acid as a starting material, and then the dehydration rate (%) was calculated by the following equation 1.

$$\text{Dehydration rate (\%)} = 100 - \text{Amount (\% by mass) of cyclohexanetetracarboxylic acid in sample} \quad \text{Equation 1}$$

(Pretreatment conditions for liquid chromatography)

First, 2 g of a sample was precisely weighed, 100 ml of dehydrated methanol was added, the mixture was heated to reflux for 1 hour to carry out a methyl esterification reaction, and thereby a liquid chromatography sample was prepared.
Provided that, in this pretreatment, the 1,2,4,5-cyclohexanetetracarboxylic acid as a reaction starting material in the sample is not esterified.

(Liquid chromatography analysis conditions)

The liquid chromatography analysis conditions were as follows.

Liquid chromatography analyzer: LC-6AD (solvent delivery unit), CTO-10A (constant temperature chamber), SCL-10A (UV), SPD-10AV (UV-VIS detector), SPD-M20A (PDA detector)
Column: Shodex RSpak DE-413L
Detector: UV (210 nm)
Eluent composition: Solution A = Acetonitrile, Solution B = 0.5% Aqueous phosphoric acid solution
Mode: Binary gradient
Flow rate: 1.0 ml/min
Temperature of constant temperature chamber: 35°C

The eluent conditions were as follows. The eluent had solution A:solution B = 10:90 (volume ratio) at an analysis time of 0 to 15 minutes, and a gradient of solution A:solution B = from 10:90 (volume ratio) to 50:50 (volume ratio) was created at 15 to 20 minutes. Moreover, a gradient of solution A:solution B = from 50:50 (volume ratio) to 80:20 (volume ratio) was created at an analysis time of 20 to 25 minutes. The ratio was retained at solution A:solution B = 80:20 (volume ratio) until at 40 minutes, then a gradient of solution A:solution B = from 80:20 (volume ratio) to 10:90 (volume ratio) was created at an analysis time of 40 to 50 minutes, and the ratio was retained at solution A:solution B = 10:90 (volume ratio) until at 70 minutes.
In the above liquid chromatography, cyclohexanetetracarboxylic acid was measured, wherein the amount of cyclohexanetetracarboxylic acid in a sample was quantified by an absolute calibration method to determine the mass proportion of cyclohexanetetracarboxylic acid in the sample, and the resulting value was subtracted from 100 to obtain a dehydration rate.
That is, when 2 g of unreacted cyclohexanetetracarboxylic acid is contained in a 100 g sample, the dehydration rate is 98%.

Industrial Applicability

As described above, according to the production method of the present invention, cyclohexanetetracarboxylic dianhydride can be stably obtained at a high dehydration rate.
The cyclohexanetetracarboxylic dianhydride obtained by the present invention has high purity and is thus expected to be used as a starting material of polyimides, epoxy resin curing agents, and solder resists.

## Claims

1. A method for producing 1,2,4,5-cyclohexanetetracarboxylic dianhydride by subjecting 1,2,4,5-cyclohexanetetracarboxylic acid to a dehydration reaction in a slurry state in the presence of a dehydrating agent, wherein

   the dehydration agent is acetic anhydride;
   the acetic anhydride is used in an amount of 2.0 to 100 moles per mole of the 1,2,4,5-cyclohexanetetracarboxylic acid; and
   the dehydration reaction of the 1,2,4,5-cyclohexanetetracarboxylic acid is carried out in the presence of the dehydrating agent and a solvent, wherein

the solvent is acetic acid,
**characterized in that** the 1,2,4,5-cyclohexanetetracarboxylic acid is fed in a continuous manner to the dehydrating agent, wherein
an average particle size of the 1,2,4,5-cyclohexanetetracarboxylic acid is less than 20 μm.

2. The method according to claim 1, wherein an average particle size of the 1,2,4,5-cyclohexanetetracarboxylic acid is less than 7 μm.

3. The method according to claim 1 or 2, wherein a reaction temperature of the dehydration reaction is 80 to 150°C.

## Patentansprüche

1. Verfahren zur Herstellung von 1,2,4,5-Cyclohexantetracabonsäure-Dianhydrid, indem 1,2,4,5-Cyclohexantetracabonsäure in aufgeschlämmtem Zustand in Gegenwart eines Dehydratisierungsmittels einer Dehydratisierungsreaktion unterzogen wird, wobei

   das Dehydratisierungsmittel Essigsäureanhydrid ist;
   das Essigsäureanhydrid in einer Menge von 2,0 bis 100 Mol pro Mol der 1,2,4,5-Cyclohexantetracabonsäure verwendet wird; und
   die Dehydratisierungsreaktion der 1,2,4,5-Cyclohexantetracabonsäure in Gegenwart des Dehydratisierungsmittels und eines Lösungsmittels durchgeführt wird, wobei
   das Lösungsmittel Essigsäure ist,
   dadurch charakterisiert, dass die 1,2,4,5-Cyclohexantetracabonsäure in kontinuierlicher Weise zu dem Dehydratisierungsmittel zugeführt wird, wobei
   eine durchschnittliche Partikelgröße der 1,2,4,5-Cyclohexantetracabonsäure weniger als 20 μm beträgt.

2. Verfahren gemäß Anspruch 1, wobei eine durchschnittliche Partikelgröße der 1,2,4,5-Cyclohexantetracabonsäure weniger als 7 μm beträgt.

3. Verfahren gemäß Anspruch 1 oder 2, wobei eine Reaktionstemperatur der Dehydratisierungsreaktion 80 bis 150°C beträgt.

## Revendications

1. Procédé de production de dianhydride de 1,2,4,5-cyclohexanetétracarboxylique en soumettant de l'acide 1,2,4,5-cyclohexanetétracarboxylique à une réaction de déshydratation à l'état de boue en présence d'un agent de déshydratation, dans lequel

   l'agent de déshydratation est un anhydride acétique ;
   l'anhydride acétique est utilisé selon une quantité allant de 2,0 à 100 moles par mole de l'acide 1,2,4,5-cyclohexanetétracarboxylique ; et
   la réaction de déshydratation de l'acide 1,2,4,5-cyclohexanetétracarboxylique est effectuée en présence de l'agent de déshydratation et d'un solvant, dans lequel
   le solvant est de l'acide acétique,
   **caractérisé en ce que** l'acide 1,2,4,5-cyclohexanetétracarboxylique est fourni d'une manière continue à l'agent de déshydratation, dans lequel
   une granulométrie moyenne de l'acide 1,2,4,5-cyclohexanetétracarboxylique est inférieure à 20 μm.

2. Procédé selon la revendication 1, dans lequel une granulométrie moyenne de l'acide 1,2,4,5-cyclohexanetétracarboxylique est inférieure à 7 μm.

3. Procédé selon la revendication 1 ou 2, dans lequel une température de réaction de la réaction de déshydratation est de 80 à 150°C.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2003286222 A
- EP 1323700 A
- JP S53103437 A
- WO 2010010869 A